# EUROPÄISCHE PATENTSCHRIFT

(11) **EP 4 089 362 B1**
(45) Veröffentlichungstag und Bekanntmachung des Hinweises auf die Patenterteilung: **30.10.2024**
(21) Anmeldenummer: 21173564.2
(22) Anmeldetag: 12.05.2021
(51) Int. Cl.: G01B 11/25, A61C 9/00, A61B 5/103, A61B 5/00, A61B 5/107

(54) **VERFAHREN ZUM ERFASSEN EINER DEMINERALISIERUNG VON ZAHNSUBSTANZ**
METHOD FOR DETECTING A DEMINERALIZATION OF TOOTH SUBSTANCE
PROCÉDÉ DE DÉTECTION D'UNE DÉMINÉRALISATION DE LA SUBSTANCE DENTAIRE

(43) Veröffentlichungstag der Anmeldung: 16.11.2022
(73) Patentinhaber: Ivoclar Vivadent AG, 9494 Schaan (LI)
(72) Erfinder: KIENLE, Alwin, 89134 Blaustein (DE); NOTHELFER, Steffen, 89278 Nersingen (DE)
(74) Vertreter: Baldus, Oliver

(56) Entgegenhaltungen:
- WO-A1-2019/055569
- WO-A1-2021/084023
- US-A1- 2017 319 057
- S. E. D. WEBB ET AL, JOURNAL OF FLUORESCENCE, vol. 12, no. 2, 1 January 2002 (2002-01-01), pages 279 - 283, XP055085493, ISSN: 1053-0509, DOI: 10.1023/A:1016890019980
- SRINIVASAN V ET AL: "AUTOMATED PHASE-MEASURING PROFILOMETRY OF 3-D DIFFUSE OBJECTS", APPLIED OPTICS, OPTICAL SOCIETY OF AMERICA, WASHINGTON, DC, US, vol. 23, no. 18, 15 September 1984 (1984-09-15), pages 3105 - 3108, XP001048504, ISSN: 0003-6935, DOI: 10.1364/AO.23.003105

## Beschreibung

Die vorliegende Erfindung betrifft ein optisches Verfahren zum Erfassen einer Demineralisierung einer Zahnsubstanz und ein Dentalgerät zum Erfassen einer Demineralisierung einer Zahnsubstanz.

Derzeitig wird Karies entweder rein visuell oder mit technischen Hilfsmitteln bestimmt, wie beispielsweise anhand von Röntgenaufnahmen oder der Autofluoreszenz kariöser Läsionen. Die visuell durchgeführte Feststellung von Karies hängt von der Erfahrung des behandelnden Zahnarztes ab und ist daher in Bezug auf Standardisierung oder Qualitätssicherung nicht ideal und objektiv quantifizierbar. Außerdem ist es nicht möglich, anhand einer Dokumentation Verfärbungen von Zähnen frühzeitig quantitativ zu ermitteln. Weitere Verfahren zur Bestimmung von Karies sind aus den Dokumenten US 2017/319057 A1, WO 2021/084023 A1, WO 2019/055569 A1 oder S. E. D. Webb et al:J. of Fluorescence Bd. 12, Nr. 2(2002-01-01), Seiten 279-283, bekannt.

Es ist die technische Aufgabe der vorliegenden Erfindung, Bereiche einer Zahnsubstanz zu bestimmen, die von einer Demineralisierung betroffen sind, wie diese bei einem Kariesbefall vorkommen kann.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Erfassen einer Demineralisierung einer Zahnsubstanz, mit den Schritten eines Einstrahlens eines strukturierten Lichtmusters auf die Zahnsubstanz; eines Erfassens einer aus dem Volumen der Zahnsubstanz remittierten Lichtintensität des Lichtmusters und/oder Intensitätsamplitude; eines Erfassens einer aus dem Volumen der Zahnsubstanz remittierten Lichtintensität des Lichtmusters; und eines Bestimmens einer Demineralisierung der Zahnsubstanz auf Basis der erfassten Lichtintensität und einer Phasenverschiebung zwischen dem eingestrahlten Lichtmuster und dem remittierten Lichtmuster. Durch das Verfahren kann eine frühzeitige Bildgebung von morphologischen oder chemischen Änderungen in der Zahnsubstanz erzielt werden. Zusätzlich kann eine Demodulation der Intensitätsamplitude durchgeführt werden. Bei dem Verfahren kann zusätzlich ein an der Zahnoberfläche reflektierte Lichtintensität erfasst werden. Anhand des Verfahrens kann die Demineralisierung objektivierbar und quantifizierbar gemacht werden und erfolgt frühzeitiger und sensitiver. Zudem wird eine Tiefensensitivität erreicht, durch die die Demineralisierung im Volumen der Zahnsubstanz bestimmt werden kann. Durch die Vergleichbarkeit und Standardisierung von gewonnenen Datensätzen wird eine Quantifizierbarkeit der Untersuchung von Initialkaries erreicht. Diese kann in objektiver Weise dokumentiert werden.

In einer technisch vorteilhaften Ausführungsform des Verfahrens wird ein Fluoreszenzübergang der remittierten Lichtintensität und/oder eine elastische Streuung der remittierten Lichtintensität erfasst. Es kann auch ein Fluoreszenzübergang der reflektierten Lichtintensität und/oder eine elastische Streuung der reflektierten Lichtintensität erfasst werden. Dadurch wird beispielsweise der technische Vorteil erreicht, dass sich eine Demineralisierung mit hoher Präzision feststellen lässt.

In einer weiteren technisch vorteilhaften Ausführungsform des Verfahrens weist das eingestrahlte strukturierte Lichtmuster ein Streifenmuster, ein Punktmuster oder ein Gittermuster und/oder eine periodische Struktur auf. Dadurch wird beispielsweise der technische Vorteil erreicht, dass sich Veränderungen in der Zahnsubstanz schnell und genau erfassen lassen.

In einer weiteren technisch vorteilhaften Ausführungsform des Verfahrens werden mehrere strukturierte Lichtmuster mit unterschiedlicher Ortsfrequenz auf die Zahnsubstanz eingestrahlt. Dadurch wird beispielsweise der technische Vorteil erreicht, dass durch eine Optimierung der Ortsfrequenz des eingestrahlten Lichtmusters, wie beispielsweise ein Sinusmuster, die Sensitivität auf den zu messenden Tiefenbereich maximiert und eine Tiefenauflösung erreicht werden kann.

In einer weiteren technisch vorteilhaften Ausführungsform des Verfahrens werden mehrere strukturierte Lichtmuster mit jeweils unterschiedlicher Lichtwellenlänge auf die Zahnsubstanz eingestrahlt. Dadurch wird beispielsweise der technische Vorteil erreicht, dass sich Daten über die Demineralisierung aus unterschiedlicher Tiefe der Zahnsubstanz gewinnen lassen sowie zusätzliche Information über die Morphologie bestimmt werden kann.

Gemäß der Erfindung wird die Demineralisierung der Zahnsubstanz zusätzlich auf Basis einer Phasenverschiebung zwischen dem eingestrahlten Lichtmuster und dem remittierten Lichtmuster bestimmt. Die Demineralisierung der Zahnsubstanz kann auch auf Basis einer Phasenverschiebung zwischen dem eingestrahlten Lichtmuster und dem reflektierten Lichtmuster bestimmt werden. Dadurch wird beispielsweise der technische Vorteil erreicht, dass sich die Demineralisierung noch genauer bestimmen lässt. In einer weiteren technisch vorteilhaften Ausführungsform des Verfahrens wird die Intensitätsamplitude und Phasenverschiebung ortsaufgelöst über einen Flächenbereich bestimmt und die Demineralisierung ortsaufgelöst quantifiziert oder auf Basis des Flächenbereichs bestimmt. Dadurch wird beispielsweise der technische Vorteil erreicht, dass die Demineralisierung insbesondere über eine Oberfläche der Zahnsubstanz robuster bestimmt werden kann.

In einer weiteren technisch vorteilhaften Ausführungsform des Verfahrens wird die Demineralisierung und deren Schichtdicke modellgestützt auf Basis der erfassten Lichtintensität bestimmt. Die Demineralisierung oder eine Schichtdicke kann modellgestützt auf Basis der erfassten Lichtintensität unter Verwendung von analytischen Gleichungen der Strahlungstransporttheorie für beispielsweise geschichtete Geometrien bestimmt werden. Dadurch wird beispielsweise der technische Vorteil erreicht, dass eine quantitative und damit vergleichbare Größe für die Charakterisierung der Demineralisierung gewonnen wird.

In einer weiteren technisch vorteilhaften Ausführungsform des Verfahrens liegt der Winkel zwischen einer Richtung, in der das Lichtmuster eingestrahlt wird, und einer Richtung, in der die Lichtintensität des Lichtmusters erfasst wird, zwischen 0° und 45°. Dadurch wird beispielsweise der technische Vorteil erreicht, dass sich die Demineralisierung besonders gut erfassen lässt, indem beispielsweise Reflexe von Oberflächen vermieden oder vermindert werden.

In einer weiteren technisch vorteilhaften Ausführungsform des Verfahrens wird zusätzlich eine räumliche Geometrie der Zahnsubstanz auf Basis des reflektierten und/oder remittierten Lichtmusters bestimmt. Dadurch wird beispielsweise der technische Vorteil erreicht, dass zusätzlich die räumliche Form des Zahns oder der Zahnsubstanz erfasst werden kann.

In einer weiteren technisch vorteilhaften Ausführungsform des Verfahrens wird die räumliche Geometrie der Zahnsubstanz modellgestützt aus mehreren reflektierten und/oder remittierten Lichtmustern mit jeweils unterschiedlichen Ortsfrequenzen bestimmt. Die ermittelte räumliche Geometrie kann verwendet werden, um die Demineralisierung der Zahnsubstanz genauer zu quantifizieren. Dadurch wird beispielsweise der technische Vorteil erreicht, dass sich die Genauigkeit der Erfassung der räumlichen Form erhöht.

Gemäß einem zweiten Aspekt wird die technische Aufgabe durch ein Dentalgerät zum Erfassen einer Demineralisierung einer Zahnsubstanz gelöst, mit einer Projektionsvorrichtung zum Einstrahlen eines strukturierten Lichtmusters auf die Zahnsubstanz; einer Erfassungsvorrichtung zum Erfassen einer aus dem Volumen der Zahnsubstanz remittierten Lichtintensität des Lichtmusters und/oder Intensitätsamplitude; und einer Bestimmungsvorrichtung zum Bestimmen einer Demineralisierung der Zahnsubstanz auf Basis der erfassten Lichtintensität und einer Phasenverschiebung zwischen dem eingestrahlten Lichtmuster und dem remittierten Lichtmuster.

Zusätzlich kann die Erfassungsvorrichtung ausgebildet sein, eine von der Oberfläche reflektierten Lichtintensität des Lichtmusters zu erfassen.

In einer technisch vorteilhaften Ausführungsform des Dentalgeräts umfasst die Projektionsvorrichtung einen Digitalprojektor mit multispektraler Strahlungsquelle. Dadurch wird beispielsweise der technische Vorteil erreicht, dass Lichtmuster mit unterschiedlicher Struktur oder Lichtmuster mit ortsaufgelöst beliebig veränderbarer Intensität unterschiedlicher Lichtwellenlänge eingestrahlt werden können. In einer weiteren technisch vorteilhaften Ausführungsform des Dentalgeräts umfasst die Erfassungsvorrichtung eine elektronische Kamera. Dadurch wird beispielsweise der technische Vorteil erreicht, dass das Lichtmuster schnell und effizient digital erfasst werden kann.

In einer weiteren technisch vorteilhaften Ausführungsform des Dentalgeräts ist die Bestimmungsvorrichtung ausgebildet, eine räumliche Geometrie der Zahnsubstanz auf Basis des remittierten und/oder reflektierten Lichtmusters zu bestimmen. Dadurch wird beispielsweise der technische Vorteil erreicht, dass zusätzlich die räumliche Form des Zahns oder der Zahnsubstanz erfasst werden kann.

Es zeigen:
- Fig. 1: eine schematische Ansicht eines Dentalgerätes;
- Fig. 2: eine schematische Ansicht unterschiedlicher Lichtmuster und Phasenverschiebungen beim Eindringen in die Zahnsubstanz;
- Fig. 3: unterschiedliche simulierte Querschnittsansichten beim Eindringen unterschiedlicher Lichtmuster in die Zahnsubstanz; und
- Fig. 4: ein Blockdiagramm des Verfahrens zum Erfassen einer Demineralisierung einer Zahnsubstanz.

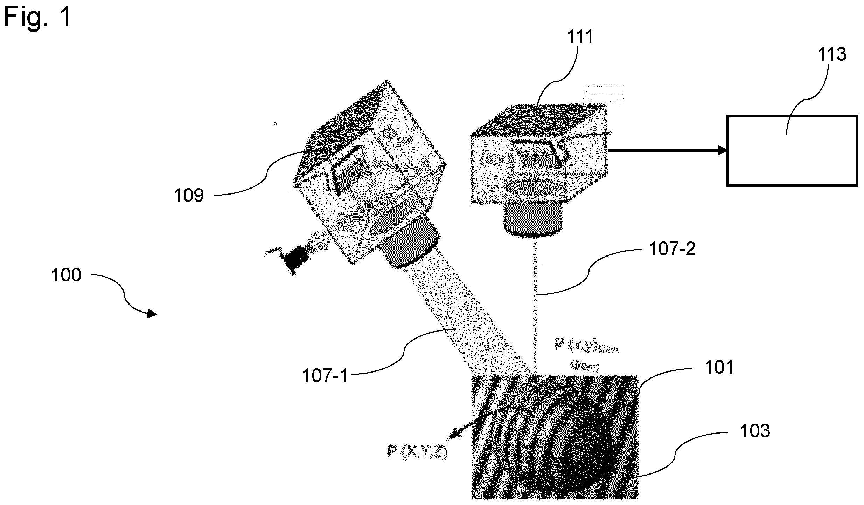

Fig. 1 zeigt eine schematische Ansicht eines Dentalgerätes 100. Das Dentalgerät 100 dient zum Erfassen einer Demineralisierung einer Zahnsubstanz 101 eines Zahnes. Das Dentalgerät 100 umfasst eine Projektionsvorrichtung 109 zum Einstrahlen eines strukturierten Lichtmusters 103 auf die Zahnsubstanz 101. Die Projektionsvorrichtung 109 ist beispielsweise durch einen Flüssigkristallprojektor oder einen DLP-Projektor (Digital Light Processing) mit einem Digital-Micromirror-Device (DMD) gebildet.

Durch eine geeignete Optik und Lichtquelle lassen sich hierdurch Lichtmuster in einer beliebigen Farbe mit einer beliebigen Struktur auf die Zahnsubstanz 101 projizieren, wie beispielsweise ein Streifenmuster, ein Punktmuster oder ein Gittermuster. Das Lichtmuster 103 kann eine veränderbare periodische Struktur aufweisen. Im Allgemeinen können aber auch andere geeignet strukturierte Lichtmuster 101 verwendet werden.

Zudem können die Lichtmuster verändert werden, so dass sich hintereinander mehrere strukturierte Lichtmuster mit unterschiedlicher Ortsfrequenz oder unterschiedlicher Lichtwellenlänge auf die Zahnsubstanz 101 einstrahlen lassen. Durch eine multispektrale Beleuchtung kann die Robustheit des Verfahrens erhöht werden. Da die Größenordnungen struktureller Veränderungen in der Zahnsubstanz 101 bei Initialkaries anfänglich klein sind, ist es vorteilhaft, eine kleine Lichtwellenlänge zu verwenden, d.h. beispielsweise blaues Licht, um eine möglichst hohe Empfindlichkeit zu erreichen.

Das strukturierte Lichtmuster 103 trifft auf die Zahnsubstanz 101 eines Zahnes in der Mundhöhle auf und dringt in die Zahnsubstanz 101 ein und wird aus dem Volumen der Zahnsubstanz101 remittiert. Dabei handelt es sich um eine diffuse (ungerichtete) Reflexion von Licht, im Gegensatz zur regulären gerichteten Reflexion, die das Reflexionsgesetz erfüllt. Ein Teil des Lichtmusters 103 kann auch an der Oberfläche der Zahnsubstanz 101 reflektiert werden.

Bei der Remission kann eine elastische Streuung des eingestrahlten Lichtmusters 101 auftreten, bei der die Wellenlänge des Lichtes unverändert bleibt, oder es tritt eine Fluoreszenzemission auf, bei der die Wellenlänge des Lichtmusters 101 durch Fluoreszenzübergänge in der Zahnsubstanz 101 verändert wird. Fluoreszenz ist die spontane Emission von Licht kurz nach der Anregung der Zahnsubstanz durch Licht. Dabei sind die emittierten Photonen in der Regel energieärmer als die vorher absorbierten. Hierbei handelt es sich daher um eine inelastische Streuung.

Eine optische Erfassungsvorrichtung 111 dient zum Erfassen der aus dem Volumen der Zahnsubstanz 101 remittierten oder an der Oberfläche reflektierten Lichtintensität des Lichtmusters 103. Die Erfassungsvorrichtung 111 umfasst beispielsweise eine digitale Kamera mit CCD- oder CMOS-Array, durch die eine Bildaufnahme des eingestrahlten Lichtmusters 101 gewonnen werden kann. Die optische Erfassungsvorrichtung 111 erzeugt einen Datensatz, der das von der Zahnsubstanz 101 remittierte und/oder reflektierte Lichtmuster wiedergibt. Je nach Anzahl verwendeter Ortsfrequenzen und Lichtwellenlängen kann die Messung beispielsweise in mehreren 100 ms durchgeführt werden. Der Winkel zwischen einer Richtung 107-1, in der das Lichtmuster 103 eingestrahlt wird, und einer Richtung 107-2, in der die Lichtintensität des Lichtmusters 103 erfasst wird, kann beispielsweise zwischen 0° und 45° liegen.

Die Fluoreszenz wird in der Erfassungsvorrichtung 111 z.B. durch Verwendung von Filtern zur Unterdrückung der elastischen Streuung durch eine CCD- oder CMOS-Kamera erfasst. In diesem Fall kann eine ortsaufgelöste und tiefenselektive Messung von Fluoreszenz im Volumen der Zahnsubstanz durchgeführt werden, wie beispielsweise eine Verteilung von Porphyrinen, was eine weitere Charakterisierung der Initialkaries ermöglicht. Hierbei wird ein Lichtmuster mit z.B. blauem oder rotem Licht eingestrahlt und die zurückgestreute Strahlung im roten oder infraroten Bereich erfasst.

Eine Bestimmungsvorrichtung 113 mit einer elektronischen Schaltung dient zum Bestimmen einer Demineralisierung der Zahnsubstanz 101 auf Basis der erfassten Lichtintensität. Die Bestimmungsvorrichtung 113 umfasst hierzu beispielsweise einen digitalen Prozessor und einen digitalen Speicher, wie beispielsweise einen wahlfreien Zugriffsspeicher (RAM-Speicher). Durch diese kann der Datensatz unter Anwendung eines Algorithmus verarbeitet werden. Dadurch, dass das Lichtmuster 103 in das Volumen der Zahnsubstanz 101 eindringt, können die Eigenschaften des Volumens, nämlich eine Demineralisierung im Falle von Karies, erfasst werden. Die Bestimmungsvorrichtung 113 bestimmt zu diesem Zweck eine Intensität (Intensitätsamplitude) und eine örtliche Phasenverschiebung des remittierten Lichtmusters gegenüber dem eingestrahlten Lichtmuster 103, oder auch zusätzlich die Wellenlängenverschiebung (Fluoreszenz). Weichen diese Werte von denjenigen Werten ab, die für gesunde Zahnsubstanz 101 erhalten werden, kann auf eine Demineralisierung geschlossen werden

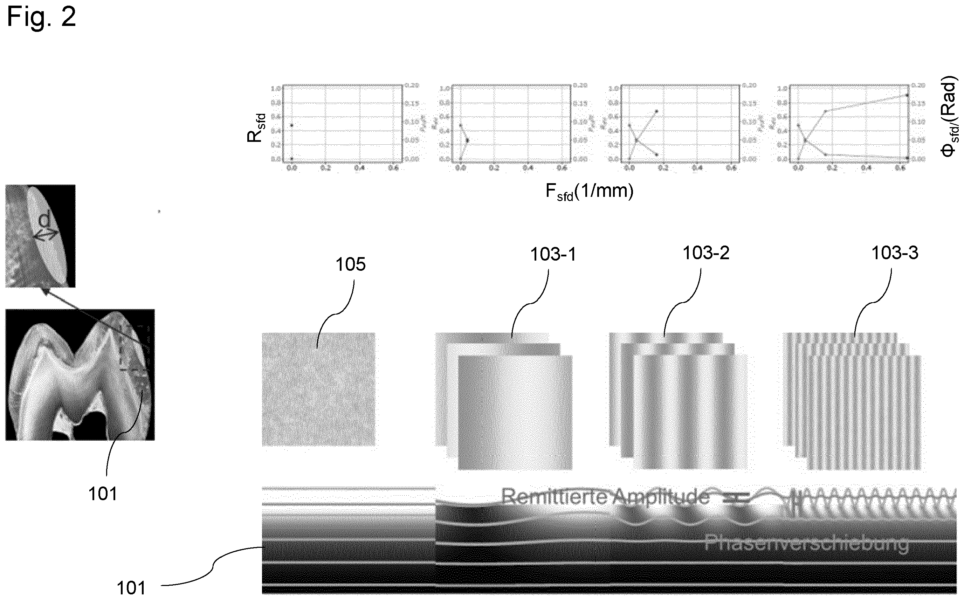

Fig. 2 zeigt unterschiedliche Lichtmuster 103-1, ... 103-3 und jeweilige Phasenverschiebungen beim Eindringen in die Zahnsubstanz 101. Bei einem homogenen Lichtmuster 105 kann keine Phasenverschiebung erfasst werden, da dieses keine Referenzstrukturen aufweist.

Das Lichtmuster 103-1 ist ein erstes Lichtmuster mit einer ersten Ortsfrequenz F_{sfd}. Dieses wird auf die Zahnsubstanz 101 eingestrahlt. Das remittierte Lichtmuster 103-1 weist gegenüber dem eingestrahlten Lichtmuster eine spezifische Phasenverschiebung φ und veränderte Intensitätsamplitude R_{sfd} auf.

Das Lichtmuster 103-2 ist ein zweites Lichtmuster mit einer zweiten Ortsfrequenz F_{sfd}, die höher als die erste Ortsfrequenz F_{sfd} ist. Das remittierte Lichtmuster 103-2 weist gegenüber dem eingestrahlten Lichtmuster ebenfalls eine spezifische Phasenverschiebung φ und veränderte Intensitätsamplitude R_{sfd} auf.

Das Lichtmuster 103-3 ist ein drittes Lichtmuster mit einer dritten Ortsfrequenz F_{sfd}, die höher als die erste und die zweite Ortsfrequenz F_{sfd} ist. Das remittierte Lichtmuster 103-3 weist gegenüber dem eingestrahlten Lichtmuster ebenfalls eine spezifische Phasenverschiebung φ und veränderte Intensitätsamplitude R_{sfd} auf. In den gezeigten Beispielen nimmt die Intensitätsamplitude R_{sfd} ab, je höher die Ortsfrequenz F_{sfd} des Lichtmusters 103 ist, und die Phasenverschiebung φ nimmt zu, je höher die Ortsfrequenz F_{sfd} des Lichtmusters 103 ist.

Je nach Demineralisierungsgrad der Zahnsubstanz werden unterschiedliche Werte für die Phasenverschiebung φ und Intensitätsamplitude R_{sfd} in Abhängigkeit der Ortsfrequenz F_{sfd} gemessen. Weichen diese Werte von denjenigen Referenzwerten ab, die für gesunde Zahnsubstanz 101 erhalten werden, kann auf eine Demineralisierung und Läsion der Zahnsubstanz 101 geschlossen werden.

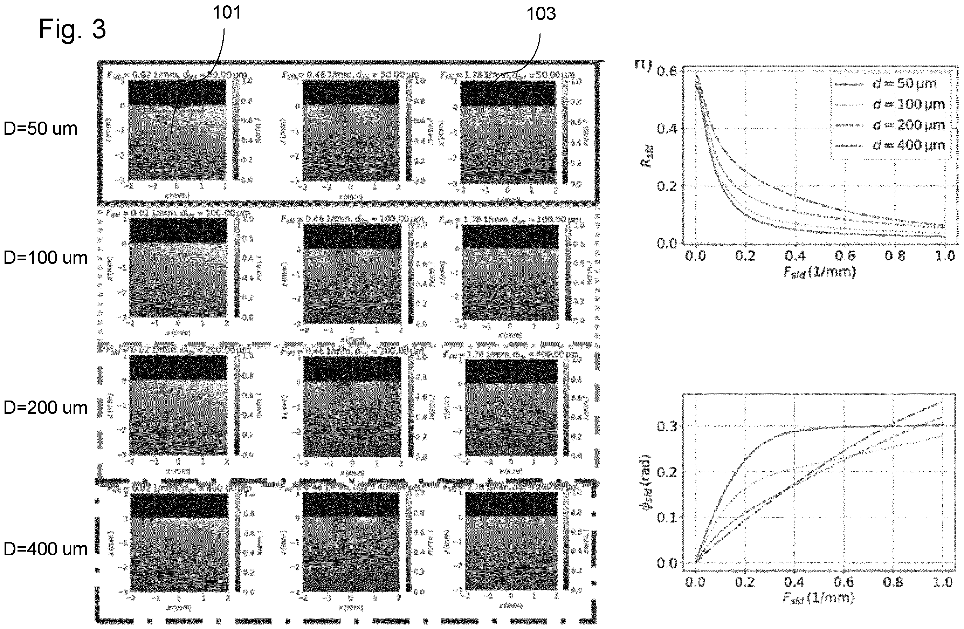

Fig. 3 zeigt unterschiedliche Querschnittsansichten beim Eindringen unterschiedlicher Lichtmuster 103 in die Zahnsubstanz 101 auf einer modellierten Läsion. Die Signale sind für unterschiedliche Dicken der Zahnsubstanz 101 dargestellt.

Die Messung des remittierten Lichts unter strukturierter Beleuchtung erlaubt die ortsaufgelöste Bestimmung der remittierten Intensitätsamplitude R_{sfd} und der Phasenverschiebung φ. Die remittierte Intensitätsamplitude R_{sfd} und Phasenverschiebung φ im Fall einer Läsion ist generell beeinflusst durch ihre optischen Eigenschaften, dem Absorptionskoeffizient µₐ, dem Streukoeffizient µₛ, der Streu-Phasenfunktion und dem Brechungsindex n, im Speziellen durch die optische Transportdicke µₛ'* d, wobei µₛ' dem effektiven Streukoeffizienten und d der Dicke der Läsion entspricht.

Bei einer Läsion mit einer größeren Dicke d ergeben sich andere Verläufe der remittierten Intensitätsamplitude R_{sfd} und der Phasenverschiebung φ in Abhängigkeit der Ortsfrequenz F_{sfd}. Die Lichtausbreitung in der Zahnsubstanz 101 ist ebenfalls abhängig von den optischen Eigenschaften (Absorptionskoeffizient µₐ, Streukoeffizient µₛ, Streu-Phasenfunktion und Brechungsindex) der Zahnsubstanz 101. Der Streukoeffizient µₛ wird durch initiale Demineralisierung relativ zum gesunden Schmelz erhöht. Eine Modellierung der Lichtausbreitung auf Basis der Strahlungstransportgleichung für geschichtete Medien ermöglicht eine Bestimmung der Dicke.

Durch eine quantitative und ortsaufgelöste Messung der remittierten und reflektierten Intensitätsamplitude und Phasenverschiebung in der Ortsfrequenzdomäne können modellgestützt durch Lösung der Strahlungstransporttheorie die für die Lichtausbreitung relevanten Größen bestimmt werden, wie beispielsweise der effektive Streukoeffizient µₛ' und der Absorptionskoeffizient µₐ. Hierbei korreliert der effektive Streukoeffizient µₛ' mit dem Grad der Demineralisierung und der Absorptionskoeffizient µₐ mit einer Verfärbung des Zahns. Beide Parameter können anhand des Verfahrens gemessen und dokumentiert werden.

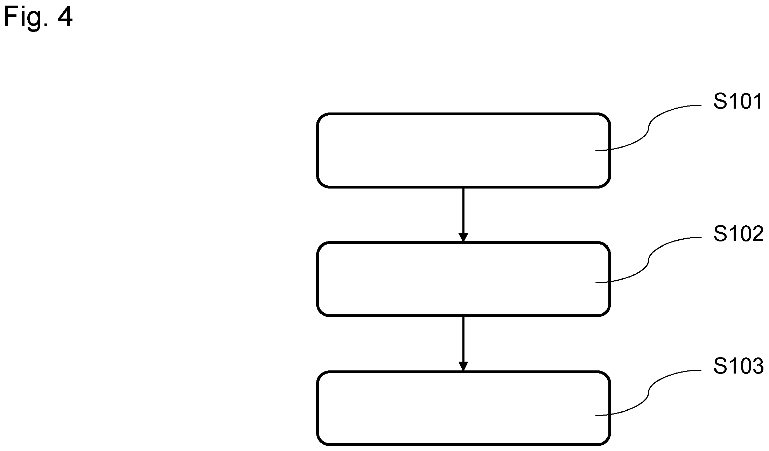

Fig. 4 zeigt ein Blockdiagramm des Verfahrens zum Erfassen einer Demineralisierung einer Zahnsubstanz 101. In Schritt S101 wird ein strukturiertes Lichtmuster 103 auf die Zahnsubstanz 101 eingestrahlt. In Schritt S102 wird die aus dem Volumen der Zahnsubstanz 101 remittierten Lichtintensität des Lichtmusters 103 erfasst. In Schritt S103 eine Demineralisierung der Zahnsubstanz 101 auf Basis der erfassten Lichtintensität bestimmt. Hierzu wird ein Vergleich der remittierten Lichtintensität mit einem Referenzwert durchgeführt. Zusätzlich zur Lichtintensität wird die Phasenverschiebung φ zwischen dem eingestrahlten Lichtmuster und dem remittierten Lichtmuster bestimmt. Auch in diesem Fall wird ein Vergleich der Phasenverschiebung φ mit einem Referenzwert durchgeführt.

Durch das Verfahren wird eine sensitive, objektive und quantitative dreidimensionale Vermessung des (De-) Mineralisierungsgrades der Zahnsubstanz 101 erfolgt. Hierzu wird beispielsweise ein Streifenlichtverfahren (Structured Illumination Imaging) so erweitert, dass auch Informationen aus der Tiefe (Volumen) der Zahnsubstanz 101 erhalten werden. Dabei kann die Zahnsubstanz 101 mit einem monochromatischen, strukturierten Lichtmuster beispielsweise im blauen Spektralbereich beleuchtet werden, wie beispielsweise einem Sinusmuster unterschiedlicher Ortsfrequenz.

Das aus dem Volumen und der Oberfläche der Zahnsubstanz 101 remittierte und reflektierte Licht wird von der Kamera erfasst. Aus dem Datensatz können durch geeignete Algorithmen die Amplituden und Phasenbilder berechnet werden, beispielsweise durch N-Phasenprojektion oder einer Fouriergestützte Demodulation. Anhand einer Kalibrierung der Kamerastrahlen in Kombination mit einer Phasencodierung durch die aktive Beleuchtung kann aus dem Kamerabild die Zahntopographie berechnet werden (3D-Scan). Zusätzlich kann unter Verwendung der rekonstruierten 3D-Zahntopographie eine volumetrische Intensitätskalibrierung durchgeführt werden, so dass neben den zuvor verwendeten Phasenbildern auch quantifizierbare Amplitudenbilder erzeugt werden können. Dadurch kann abhängig von der eingestrahlten Ortsfrequenz, d.h. der Streifenfrequenz, in jedem Bildpunkt die remittierte und reflektierte Intensitätsamplitude bestimmt werden. Dies entspricht der optischen Übertragungsfunktion der Zahnsubstanz 101, die von der Mikrostruktur abhängt. Die Mikrostruktur verursacht die Lichtstreuung und, zusätzlich zu den Chromophoren, die Zahnfärbung der Zahnsubstanz 101. Da im Falle von Karies die Zahnsubstanz 101 poröser wird, kann diese durch eine veränderte Lichtstreuung nachgewiesen werden. Eine Veränderung und Tiefe der Mikrostruktur kann modellgestützt anhand der optischen Übertragungsfunktion quantifiziert werden.

Die Demineralisierung auf Grundlage dieses Datensatzes kann entweder modellgestützt oder anhand von KI-getriebenen Verfahren oder multivarianten Klassifizierungsverfahren erfolgen. Für diese Verfahren wird zur Erstellung eines Klassifikators eine modellgestützte Berechnung der optischen Übertragungsfunktion durchgeführt. Das Verfahren ermöglicht eine quantifizierbare Messung der remittierten Intensitätsamplitude und Reflektanz an einer beliebigen Geometrie der Zahnsubstanz 101.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentalgerät
- 101: Zahnsubstanz
- 103: Lichtmuster
- 105: Homogene Beleuchtung
- 107: Richtung
- 109: Projektionsvorrichtung
- 111: Erfassungsvorrichtung
- 113: Bestimmungsvorrichtung

## Patentansprüche

1. Verfahren zum Erfassen einer Demineralisierung einer Zahnsubstanz (101), mit den Schritten:
- Einstrahlen (S101) eines strukturierten Lichtmusters (103) auf die Zahnsubstanz (101);
- Erfassen (S102) einer aus dem Volumen der Zahnsubstanz (101) remittierten Lichtintensität des Lichtmusters (103) und/oder Intensitätsamplitude (R_{sfd}); und
- Bestimmen (S103) einer Demineralisierung der Zahnsubstanz (101) auf Basis der erfassten Lichtintensität und einer Phasenverschiebung (ϕ_{sfd}) zwischen dem eingestrahlten Lichtmuster (103) und dem remittierten Lichtmuster (103).

2. Verfahren nach Anspruch 1, wobei ein Fluoreszenzübergang der remittierten Lichtintensität erfasst wird und/oder eine elastische Streuung der remittierten Lichtintensität erfasst wird.

3. Verfahren nach einem der vorangehenden Ansprüche, wobei das eingestrahlte strukturierte Lichtmuster ein Streifenmuster, ein Punktmuster oder ein Gittermuster und/oder eine periodische Struktur aufweist.

4. Verfahren nach einem der vorangehenden Ansprüche, wobei mehrere strukturierte Lichtmuster mit unterschiedlicher Ortsfrequenz auf die Zahnsubstanz (101) eingestrahlt werden.

5. Verfahren nach einem der vorangehenden Ansprüche, wobei mehrere strukturierte Lichtmuster mit jeweils unterschiedlicher Lichtwellenlänge auf die Zahnsubstanz (101) eingestrahlt werden.

6. Verfahren nach Anspruch 1, wobei die Intensitätsamplitude , R_{sfd}, und Phasenverschiebung, ϕ_{sfd}, ortsaufgelöst über einen Flächenbereich bestimmt und die Demineralisierung ortsaufgelöst quantifiziert oder auf Basis des Flächenbereichs bestimmt wird.

7. Verfahren nach einem der vorangehenden Ansprüche, wobei die Demineralisierung und eine Schichtdicke modellgestützt auf Basis der erfassten Lichtintensität bestimmt werden.

8. Verfahren nach einem der vorangehenden Ansprüche, wobei der Winkel zwischen einer Richtung (107-1), in der das Lichtmuster (103) eingestrahlt wird, und einer Richtung (107-2), in der die Lichtintensität des Lichtmusters erfasst wird, zwischen 0° und 45° liegt.

9. Verfahren nach einem der vorangehenden Ansprüche, wobei zusätzlich eine räumliche Geometrie der Zahnsubstanz (101) auf Basis des reflektierten und/oder remittierten Lichtmusters (103) bestimmt wird.

10. Verfahren nach Anspruch 9, wobei die räumliche Geometrie der Zahnsubstanz (101) modellgestützt aus mehreren reflektierten und/oder remittierten Lichtmustern (103) mit jeweils unterschiedlichen Ortsfrequenzen bestimmt wird.

11. Dentalgerät (100) zum Erfassen einer Demineralisierung einer Zahnsubstanz (101), mit:
- einer Projektionsvorrichtung (109) zum Einstrahlen eines strukturierten Lichtmusters (103) auf die Zahnsubstanz (101);
- einer Erfassungsvorrichtung (111) zum Erfassen einer aus dem Volumen der Zahnsubstanz (101) remittierten Lichtintensität des Lichtmusters (103) und/oder Intensitätsamplitude , R_{sfd}; und
- einer Bestimmungsvorrichtung (113) zum Bestimmen einer Demineralisierung der Zahnsubstanz (101) auf Basis der erfassten Lichtintensität und einer Phasenverschiebung , ϕ_{sfd}, zwischen dem eingestrahlten Lichtmuster (103) und dem remittierten Lichtmuster (103).

12. Dentalgerät (100) nach Anspruch 11, wobei die Projektionsvorrichtung (109) einen Digitalprojektor mit multispektraler Strahlungsquelle umfasst.

13. Dentalgerät (100) nach Anspruch 11 oder 12, wobei die Erfassungsvorrichtung (111) eine elektronische Kamera umfasst.

14. Dentalgerät (100) nach einem der Ansprüche 11 bis 13, wobei die Bestimmungsvorrichtung (113) ausgebildet ist, eine räumliche Geometrie der Zahnsubstanz (101) auf Basis des remittierten und/oder reflektierten Lichtmusters (103) zu bestimmen.

## Claims

1. A method of detecting demineralization of a tooth substance (101), comprising the steps of:
- irradiating (S101) a structured light pattern (103) onto the tooth substance (101);
- detecting (S102) a light intensity of the light pattern (103) and/or intensity amplitude (R_{sfd}) remitted from the volume of the tooth substance (101); and
- determining (S103) demineralization of the tooth substance (101) based on the detected light intensity and a phase shift (ϕ_{sfd}) between the irradiated light pattern (103) and the remitted light pattern (103).

2. The method according to claim 1, wherein a fluorescence transition of the remitted light intensity is detected and/or an elastic scattering of the remitted light intensity is detected.

3. The method according to any one of the preceding claims,
wherein the irradiated structured light pattern comprises a stripe pattern, a dot pattern or a grid pattern and/or a periodic structure.

4. The method according to any one of the preceding claims,
wherein several structured light patterns with different spatial frequencies are irradiated onto the tooth substance (101).

5. The method according to any one of the preceding claims,
wherein several structured light patterns, each having a different wavelength of light, are irradiated onto the tooth substance (101).

6. The method according to claim 1, wherein the intensity amplitude (R_{sfd}) and phase shift (ϕ_{sfd}) are determined spatially resolved over a surface area and the demineralization is quantified spatially resolved or determined based on the surface area.

7. The method according to any one of the preceding claims, wherein the demineralization and a layer thickness are determined in a model-based manner based on the detected light intensity.

8. The method according to any one of the preceding claims, wherein the angle between a direction (107-1) in which the light pattern (103) is irradiated and a direction (107-2) in which the light intensity of the light pattern is detected is between 0° and 45°.

9. The method according to any one of the preceding claims, wherein a spatial geometry of the tooth substance (101) is additionally determined based on the reflected and/or remitted light pattern (103).

10. The method according to claim 9, wherein the spatial geometry of the tooth substance (101) is determined in a model-based manner from several reflected and/or remitted light patterns (103) each having a different spatial frequency.

11. A dental device (100) for detecting demineralization of a tooth substance (101), comprising:
- a projection device (109) for irradiating a structured light pattern (103) onto the tooth substance (101);
- a detection device (111) for detecting a light intensity of the light pattern (103) and/or intensity amplitude, R_{sfd}, remitted from the volume of the tooth substance (101); and
- a determination device (113) for determining demineralization of the tooth substance (101) based on the detected light intensity and a phase shift, ϕ_{sfd}, between the irradiated light pattern (103) and the remitted light pattern (103).

12. The dental device (100) according to claim 11, wherein the projection device (109) comprises a digital projector having a multispectral radiation source.

13. The dental device (100) according to claim 11 or 12,
wherein the detection device (111) comprises an electronic camera.

14. The dental device (100) according to any one of claims 11 to 13, wherein the determination device (113) is configured to determine a spatial geometry of the tooth substance (101) based on the remitted and/or reflected light pattern (103).

## Revendications

1. Procédé de détection de la déminéralisation d'une substance dentaire (101), comprenant les étapes suivantes :
- irradiation (S101) d'un motif lumineux (103) structuré sur la substance dentaire (101) ;
- détection (S102) d'une intensité lumineuse du motif lumineux (103) et/ou d'une amplitude d'intensité (R_{sfd}) réémise à partir du volume de la substance dentaire (101) ; et
- détermination (S103) d'une déminéralisation de la substance dentaire (101) sur la base de l'intensité lumineuse détectée et d'un déphasage (ϕ_{sfd}) entre le motif lumineux (103) irradié et le motif lumineux (103) réémis.

2. Procédé selon la revendication 1, où une transition de fluorescence de l'intensité lumineuse émise est détectée et/ou une diffusion élastique de l'intensité lumineuse émise est détectée.

3. Procédé selon l'une des revendications précédentes, où le motif lumineux structuré irradié ayant un motif de rayures, un motif de points ou un motif en treillis et/ou une structure périodique.

4. Procédé selon l'une des revendications précédentes, où plusieurs motifs lumineux structurés de fréquences spatiales différentes sont irradiés sur la substance dentaire (101).

5. Procédé selon l'une quelconque des revendications précédentes, où plusieurs motifs lumineux structurés, ayant chacun une longueur d'onde lumineuse différente, sont irradiés sur la substance dentaire (101).

6. Procédé selon la revendication 1, où l'amplitude d'intensité, le R_{sfd} et le déphasage ϕ_{sfd} sont déterminés avec une résolution locale sur une zone de surface et la déminéralisation est quantifiée avec une résolution locale ou déterminée sur la base de la zone de surface.

7. Procédé selon l'une des revendications précédentes, où la déminéralisation et une épaisseur de couche sont déterminées à l'aide d'un modèle sur la base de l'intensité lumineuse détectée.

8. Procédé selon l'une des revendications précédentes, où l'angle entre une direction (107-1) dans laquelle le motif lumineux (103) est irradié et une direction (107-2) dans laquelle l'intensité lumineuse du motif lumineux est détectée est compris entre 0° et 45°.

9. Procédé selon l'une des revendications précédentes, où une géométrie spatiale de la substance dentaire (101) est déterminée en outre sur la base du motif lumineux (103) réfléchi et/ou réémis.

10. Procédé selon la revendication 9, où la géométrie spatiale de la substance dentaire (101) est déterminée à l'aide d'un modèle à partir de plusieurs motifs lumineux (103) réfléchis et/ou réémis, ayant chacun des fréquences spatiales différentes.

11. Dispositif dentaire (100) pour détecter la déminéralisation d'une substance dentaire (101), avec
- un dispositif de projection (109) pour irradier un motif lumineux (103) structuré sur la substance dentaire (101) ;
- un dispositif de détection (111) pour détecter une intensité lumineuse du motif lumineux (103) et/ou une amplitude d'intensité R_{sfd} réémise à partir du volume de la substance dentaire (101), et
- un dispositif de détermination (113) permettant de déterminer une déminéralisation de la substance dentaire (101) sur la base de l'intensité lumineuse détectée et d'un déphasage ϕ_{sfd} entre le motif lumineux irradié (103) et le motif lumineux (103) réémis.

12. Appareil dentaire (100) selon la revendication 11, où le dispositif de projection (109) comprend un projecteur numérique avec une source de rayonnement multispectral.

13. Appareil dentaire (100) selon la revendication 11 ou 12, où le dispositif de détection (111) comprend une caméra électronique.

14. Dispositif dentaire (100) selon l'une des revendications 11 à 13, où le dispositif de détermination (113) est conçu pour déterminer une géométrie spatiale de la substance dentaire (101) sur la base du motif lumineux (103) réémis et/ou réfléchi.
